# EUROPEAN PATENT APPLICATION

(11) **EP 3 685 869 A1**
(43) Date of publication of application: **29.07.2020**
(21) Application number: 19205401.3
(22) Date of filing: 25.10.2019
(51) Int. Cl.: A61M 5/20, A61M 5/50, A61B 5/1473, A61M 5/32

(54) **SYSTEM TO INJECT MEDICINE**

(30) Priority: 24.01.2019 KR 20190009209
(71) Applicant: Philosys Co., Ltd., Gunsan-si, Jeollabuk-do 54172 (KR)
(72) Inventor: KIM, Jong Jin, 16356 Gyeonggi-do (KR); CHOI, Woo Hyek, 17077 Gyeonggi-do (KR); KIM, Dong Woo, 07446 Seoul (KR); LEE, Won Ju, 15071 Gyeonggi-do (KR)
(74) Representative: DREISS Patentanwälte PartG mbB

(57) **Abstract**

A medicine injection device includes a header including a first connector configured to receive an electrical signal associated with a target analyte from a needle and configured to be connected to the needle, a medicine storage configured to be connected to the needle when the needle and the header are connected and store a medicine, a driver configured to apply an external force to the medicine storage, a controller configured to control the driver to measure an amount of the target analyte based on the received electrical signal, determine an injection amount in response to the measuring being completed, and inject the determined injection amount, and a notifier configured to receive, from the controller, measurement information associated with the amount of the target analyte and injection information associated with the injection amount and provide a user with the received measurement information and injection information.

## Description

### CROSS-REFERENCE TO RELATED APPLICATION(S)

This application claims the priority benefit of Korean Patent Application No. 10-2019-0009209 filed on January 24, 2019, in the Korean Intellectual Property Office, the disclosure of which is incorporated herein by reference for all purposes.

### BACKGROUND

### 1. Field

One or more example embodiments relate to medicine injecting technology.

### 2. Description of Related Art

An insulin-dependent patient needs to measure blood glucose at specific times, for example, on an empty stomach, after lunch, before dinner, after dinner, and before bed, through self-measurement (or self-monitoring) of blood glucose. When, as a result of the self-measurement, the patient needs to regulate his/her blood glucose, the patient may control the blood glucose by determining an optimal amount of insulin and injecting the determined amount of insulin himself/herself using an insulin syringe, for example.

The self-measurement may require, for example, a blood glucose self-measurement device, an insulin cartridge, an insulin injector, and the like. In addition, an insulin-dependent patient may need to record a measured blood glucose level and an accurate injected amount of insulin, and provide such records to a medical practitioner or specialist to receive feedback.

For example, an insulin-dependent patient may verify a current blood glucose level using the blood glucose self-measurement device by puncturing skin with a lancet and collecting a drop of blood on a test strip. When, as a result of the verifying through the blood glucose self-measurement device, the patient needs to regulate the blood glucose level, the patient may need to prepare the insulin injector. The patient may then need to manually set, in the insulin injector, an amount of insulin to be injected that is recommended by a medical practitioner or a doctor. The patient may then need to place the insulin injector on a portion of the skin at which the insulin is to be injected, and manually inject the insulin. In addition, the patient may need to record the measured blood glucose level and the amount of injected insulin himself/herself in a blood glucose regulation recording sheet, for example.

### SUMMARY

An aspect of the present disclosure provides a medicine injection device that may measure a blood glucose level and inject insulin simultaneously.

The medicine injection device may inject a medicine stably and accurately.

The medicine injection device may inject an optimal amount of a medicine without a need to visit a hospital or clinic.

According to an aspect, there is provided a medicine injection device. The medicine injection device includes a header including a first connector configured to receive an electrical signal associated with a target analyte from a needle and configured to be connected to the needle, a medicine storage configured to be connected to the needle when the needle and the header are connected to each other, and store a medicine, a driver configured to apply an external force to the medicine storage, a controller configured to control the driver to measure an amount of the target analyte based on the electrical signal received through the first connector, determine an injection amount of the medicine to be injected in response to the measuring being completed, and inject the determined injection amount of the medicine, and a notifier configured to receive, from the controller, measurement information associated with the amount of the target analyte and injection information associated with the injection amount, and provide a user with the received measurement information and injection information.

The first connector may establish an electrical connection between the needle and the medicine injection device as the first connector is connected to a second connector disposed on an outer surface of the needle when the header and the needle are connected to each other, and be disposed on an outer surface of the header.

The medicine storage may accommodate a cartridge configured to store the medicine. The driver may apply the external force to a pusher of the cartridge accommodated in the medicine storage.

The driver may include a presser configured to apply the external force to a front end of the medicine injection device from a back end of the medicine injection device along a longitudinal axis of the medicine injection device, and a discharge regulator configured to regulate, based on the determined injection amount, a discharge amount to be discharged to the needle connected to the header from the medicine storage.

The driver may move a piston of the cartridge accommodated in the medicine storage from the back end of the medicine injection device to the front end of the medicine injection device by a distance corresponding to the determined injection amount, under the control of the controller.

The needle may include an invasive suction member configured to invade skin and absorb a body fluid sample, an invasive injection member configured to invade the skin and discharge the medicine, and a second fastener configured to support the invasive suction member and the invasive injection member, and be connected to the header.

The invasive suction member may include a biosensor configured to sense the target analyte included in the body fluid sample. When the invasive suction member includes the biosensor, the invasive injection member may be connected to the medicine storage when the header and the needle are connected to each other and discharge the medicine to be transferred from the medicine storage, and the second fastener may include a second connector disposed on an outer surface of the second fastener to be connectable to the first connector and configured to be electrically connected to the biosensor.

The second fastener may include a sensing portion including the biosensor configured to sense the target analyte included in the body fluid sample absorbed through the invasive suction member, and the second connector disposed on the outer surface of the second fastener to be connectable to the first connector and configured to be electrically connected to the biosensor.

At least one of the invasive suction member or the invasive injection member may be configured to be slidable along one axis with respect to the second fastener.

A second length by which the invasive suction member protrudes from the second fastener may be greater than a first length by which the invasive injection member protrudes from the second fastener.

When the needle invades skin, the controller may generate the measurement information by measuring an amount of the target analyte from the body fluid sample collected through the needle. When the measuring is completed, the controller may transfer the measurement information to a cloud server through a communicator. The controller may then receive the injection information corresponding to the measurement information from the cloud server, and determine the injection amount based on the received injection information.

The injection information may be calculated from the measurement information by the cloud server based on a medicine dosage estimation model.

The controller may verify whether the needle connected to the medicine injection device is previously used. When the needle is verified to be previously used, the controller may prevent an injection using the needle.

When a state of charge (SOC) of a battery is less than or equal to a reference threshold, the controller may provide the user with information that is an available number of injections through the notifier using at least one of a sound signal, a visual signal, a vibration signal, or a tactile signal, and reserve backup power of the SOC for at least one injection.

When the SOC of the battery exceeds the reference threshold while external power is being supplied through a power source socket of the medicine injection device, the controller may allow an injection during charging of the battery.

When the SOC of the battery is less than or equal to the reference threshold and exceeds a minimum threshold while external power is being supplied through the power source socket of the medicine injection device, the controller may allow an injection during charging of the battery.

The medicine injection device may further include a temperature measurer disposed separate from the battery, and configured to measure a temperature.

The medicine injection device may further include a housing configured to accommodate the medicine storage, the driver, the controller, and the notifier, and configured to be connected to the needle through the header.

According to another aspect, there is provided a medicine injection method. The medicine injection method includes receiving an electrical signal associated with a target analyte from a needle during an invasion of the needle into skin, measuring an amount of the target analyte based on the received electrical signal, determining an injection amount of a medicine to be injected in response to the measuring being completed, injecting the determined injection amount using an external force applied to a medicine storage during the invasion, and providing a user with measurement information associated with the amount of the target analyte and injection information associated with the injection amount.

Additional aspects of example embodiments will be set forth in part in the description which follows and, in part, will be apparent from the description, or may be learned by practice of the disclosure.

### BRIEF DESCRIPTION OF THE DRAWINGS

These and/or other aspects, features, and advantages of the present disclosure will become apparent and more readily appreciated from the following description of example embodiments, taken in conjunction with the accompanying drawings of which:
FIG. 1 is a perspective view illustrating an example of a medicine injection device according to an example embodiment;
FIG. 2 is a diagram illustrating an example of a configuration of a medicine injection device according to an example embodiment;
FIGS. 3 and 4 are diagrams illustrating examples of a configuration of a needle according to an example embodiment;
FIGS. 5 and 6 are diagrams illustrating examples of a configuration of a driver according to an example embodiment;
FIG. 7 is a diagram illustrating an example of an additional configuration of a medicine injection device according to an example embodiment;
FIG. 8 is a flowchart illustrating an example of a medicine injection method according to an example embodiment; and
FIG. 9 is a diagram illustrating an example of a medicine injection method according to an example embodiment.

### DETAILED DESCRIPTION

The following detailed description is provided to assist the reader in gaining a comprehensive understanding of the methods, apparatuses, and/or systems described herein. However, various changes, modifications, and equivalents of the methods, apparatuses, and/or systems described herein will be apparent after an understanding of the disclosure of this application. For example, the sequences of operations described herein are merely examples, and are not limited to those set forth herein, but may be changed as will be apparent after an understanding of the disclosure of this application, with the exception of operations necessarily occurring in a certain order. Also, descriptions of features that are known in the art may be omitted for increased clarity and conciseness.

The features described herein may be embodied in different forms and are not to be construed as being limited to the examples described herein. Rather, the examples described herein have been provided merely to illustrate some of the many possible ways of implementing the methods, apparatuses, and/or systems described herein that will be apparent after an understanding of the disclosure of this application.

The terminology used herein is for describing various examples only and is not to be used to limit the disclosure. As used herein, the articles "a," "an," and "the" are intended to include the plural forms as well, unless the context clearly indicates otherwise. The terms "comprises," "includes," and "has" specify the presence of stated features, numbers, operations, members, elements, and/or combinations thereof, but do not preclude the presence or addition of one or more other features, numbers, operations, members, elements, and/or combinations thereof.

Although terms such as "first," "second," "third," A, B, (a), (b), and the like may be used herein to describe various members, components, regions, layers, or sections, these members, components, regions, layers, or sections are not to be limited by these terms. Rather, these terms are only used to distinguish one member, component, region, layer, or section from another member, component, region, layer, or section. Thus, a first member, component, region, layer, or section referred to in examples described herein may also be referred to as a second member, component, region, layer, or section without departing from the teachings of the examples.

It should be noted that if it is described in the specification that one component is "connected," "coupled," or "joined" to another component, a third component may be "connected," "coupled," and "joined" between the first and second components, although the first component may be directly connected, coupled or joined to the second component. In addition, it should be noted that if it is described in the specification that one component is "directly connected" or "directly joined" to another component, a third component may not be present therebetween. Likewise, expressions, for example, "between" and "immediately between" and "adjacent to" and "immediately adjacent to" may also be construed as described in the foregoing. As used herein, the term "and/or" includes any one and any combination of any two or more of the associated listed items.

Unless otherwise defined, all terms, including technical and scientific terms, used herein have the same meaning as commonly understood by one of ordinary skill in the art to which this disclosure pertains based on an understanding of the present disclosure. Terms, such as those defined in commonly used dictionaries, are to be interpreted as having a meaning that is consistent with their meaning in the context of the relevant art and the present disclosure, and are not to be interpreted in an idealized or overly formal sense unless expressly so defined herein.

Also, in the description of example embodiments, detailed description of structures or functions that are thereby known after an understanding of the disclosure of the present application will be omitted when it is deemed that such description will cause ambiguous interpretation of the example embodiments.

Hereinafter, examples will be described in detail with reference to the accompanying drawings, and like reference numerals in the drawings refer to like elements throughout.

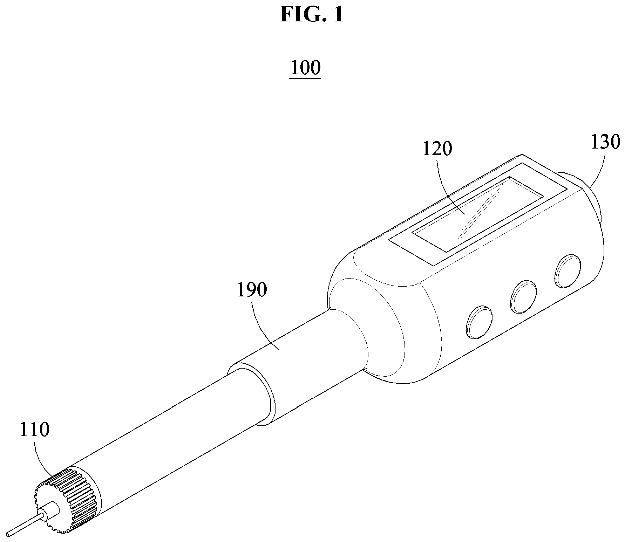

FIG. 1 is a perspective view illustrating an example of a medicine injection device according to an example embodiment.

According to an example embodiment, a medicine injection device 100 may sense an amount of a target biological material and inject an amount of a medicine corresponding to the sensed amount, through a single invasion. A biological material described herein refers to a material associated with a living body, and may also be referred herein as an analyte. The target analyte may be, for example, blood glucose, but not limited thereto. For example, when the target analyte is blood glucose, a corresponding medicine may be insulin.

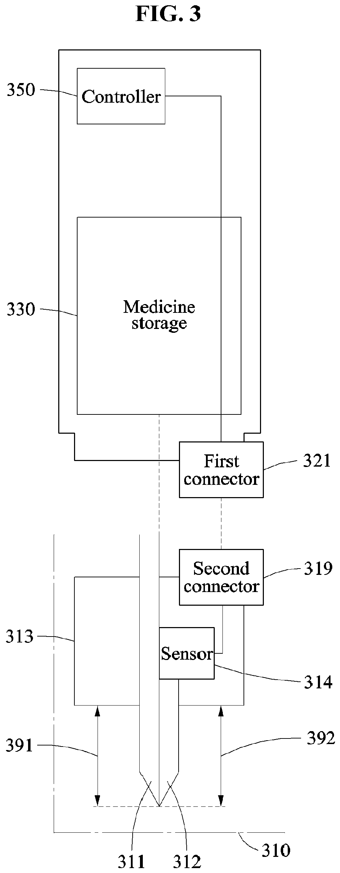

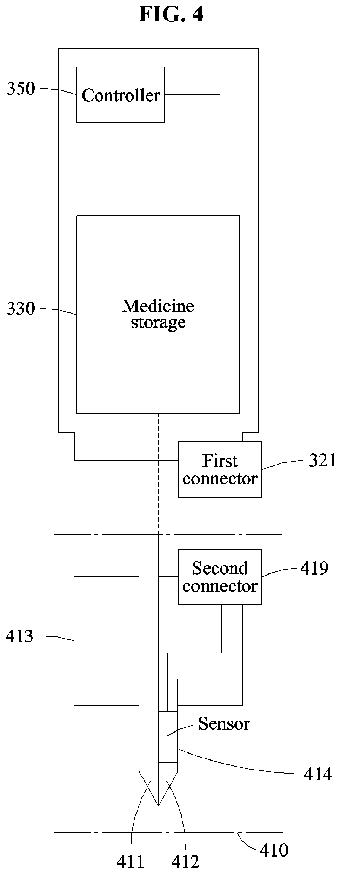

The medicine injection device 100 collects a body fluid sample of a living body through a needle 110, and senses an amount of a target analyte. The medicine injection device 100 also injects a medicine through the needle 110. The needle 110 includes an invasive member configured to invade skin. The needle 110 will be described in greater detail with reference to FIGS. 3 and 4.

The medicine injection device 100 includes a housing 190. The housing 190 supports the needle 110 and a cartridge. In the housing 190, an output interface 120 configured to provide various sets of information, and an input interface 130 configured to receive an input or instruction from a user are disposed.

The output interface 120 includes, for example, a display configured to provide visual information, and a speaker configured to provide sound information. The input interface 130 includes, for example, a button configured to operate by a control action of the user. The output interface 120 and the input interface 130 illustrated in FIG. 1 are provided as examples, and thus types of such interface are limited to the illustrated examples.

In an example, the medicine injection device 100 may detect an invasion of the needle 110 into skin, and start to measure an amount of a target analyte in response to the invasion being detected. When the measuring of the amount of the target analyte is completed, the medicine injection device 100 may determine an amount of a medicine to be injected, or an injection amount hereinafter. The medicine injection device 100 may automatically inject the determined injection amount, but not limited thereto. The medicine injection device 100 may detect an injection signal that is generated by a manipulation of the user, for example, an act of pushing a button of an injection inputter, or an injection button. For example, when the injection button is pushed by the user, a valid injection signal may be generated. While the injection signal is being detected, the medicine injection device 100 may inject the determined injection amount of the medicine. However, when the injection signal is disconnected or interrupted while the determined injection amount of the medicine is being injected, for example, when a portion of the injection amount of the medicine is injected, the medicine injection device 100 may stop injecting the medicine. In addition, when the injection amount of the medicine is all injected, the medicine injection device 100 may terminate injecting the medicine even though the injection signal is detected. Thus, the medicine injection device 100 may safely inject an injection amount of a medicine that is automatically determined based on a measured amount of a target analyte, under the control of a user. The injection inputter described above is not limited to a mechanical button switch, and may thus be embodied by an electronic switch such as a touch switch, for example.

In another example, after the automatically determined injection amount of the medicine is all injected, the medicine injection device 100 may further inject an additional amount of the medicine under the control of the user. For example, in response to an additional injection instruction or command being input from the user after the automatically determined injection amount of the medicine is injected, the medicine injection device 100 may further inject the additional amount of the medicine. The medicine injection device 100 may additionally inject the medicine while a valid additional injection instruction or command is being received from the user. When the additional injection instruction is canceled, the medicine injection device 100 may suspend or stop additionally injecting the medicine. In addition, the medicine injection device 100 may limit, to a maximum threshold amount, the additional amount of the medicine to be injected by the additional injection instruction. The maximum threshold amount may be a maximum upper limit that is to be additionally injected after the automatic injection. For example, when the medicine injection device 100 receives the additional injection instruction, the medicine injection device 100 may additionally inject the medicine up to the maximum threshold amount. However, when the maximum threshold amount of the medicine is injected, the medicine injection device 100 may terminate injecting the medicine even though the additional injection instruction continues being input.

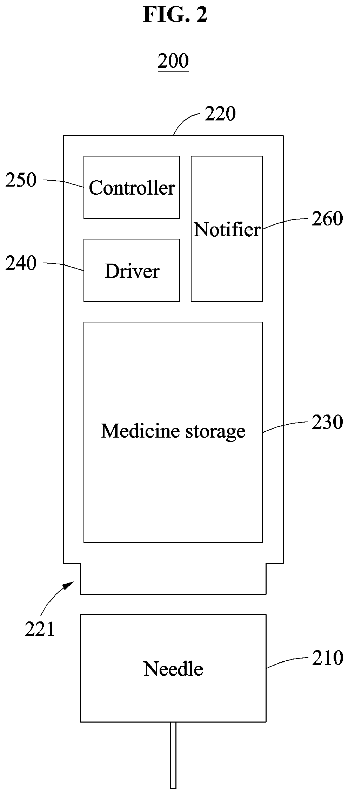

FIG. 2 is a diagram illustrating an example of a configuration of a medicine injection device according to an example embodiment.

Referring to FIG. 2, a medicine injection device 200 includes a housing 220, a medicine storage 230, a driver 240, a controller 250, and a notifier 260.

A header 221 of the housing 220 is configured to be connected to a needle 210. The header 221 includes a first fastener configured to be connected to the needle 210. Thus, the medicine injection device 200 is configured to be connected to the needle 210 through the first fastener. The first fastener is configured to be connected to a second fastener of the needle 210, and may be embodied in a fastening structure such as a screw structure and a magnetic structure. For example, one of the first fastener and the second fastener may include a thread structure and the other may include a thread groove structure. In this example, the thread structure and the thread groove structure may be engaged with each other. For another example, magnets disposed in the first fastener and the second fastener may have different magnetic properties, and each of the magnets may be embodied by a permanent magnet or an electromagnet. In addition, the header 221 of the housing 220 includes a first connector configured to receive an electrical signal associated with a target analyte from the needle 210, and the first connector is configured to be connected to a second connector of the needle 210. The first connector and the second connector are configured to be electrically connected to each other when the header 221 and the needle 210 are connected to each other. An arrangement of the first connector and the second connector will be described hereinafter with reference to FIGS. 3 and 4.

The header 221 is configured to automatically separate therefrom the needle 210 that is used once to measure the target analyte and inject a medicine. For example, the header 221 may receive an error code from the needle 210 that is used once through a connector, for example. When the error code is received, the controller 250 of the medicine injection device 200 may separate the needle 210 from the header 221. However, examples are not limited thereto. For example, when the controller 250 receives the error code indicating that the needle 210 is already used once, the controller 250 may only prevent a measuring operation and an injecting operation.

The housing 220 is configured to support the medicine storage 230, the driver 240, the controller 250, and the notifier 260. The housing 220 may be embodied in a form of a pen having a length sufficiently greater than a width thereof, or in a form of a small box of which a difference between a length and a width is less than a threshold length. The form of the housing 220 may vary depending on a form of the medicine storage 230, the driver 240, the controller 250, and the notifier 260 that are embedded therein.

The medicine storage 230 is configured to store a medicine, and accommodate a cartridge configured to store the medicine. When the needle 210 and the header 221 of the medicine injection device 200 are connected to each other, the medicine storage 230 is configured to be connected to the needle 210 to form a medicine transfer path. For example, a front end of the needle 210 is exposed from a fastener of the needle 210 to extend, and a back end of the needle 210 penetrates through the header 221 of the housing 210 to be directly or indirectly connected to the medicine storage 230. The medicine storage 230 is configured to transfer the medicine to the needle 210. For example, the cartridge is attachable to or detachable from the medicine storage 230, and thus a user may mount, in the medicine injection device 230, the cartridge according to a prescription by a doctor or a medical practitioner.

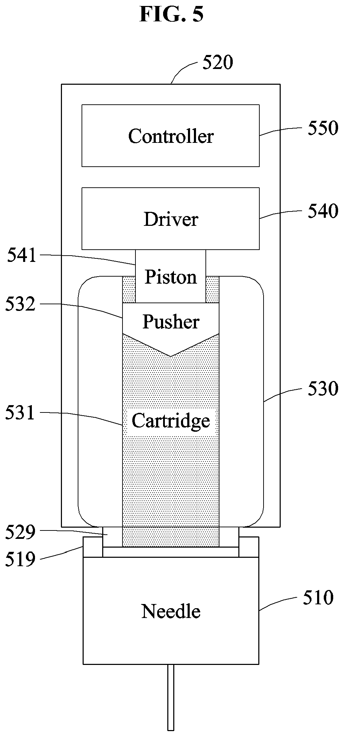

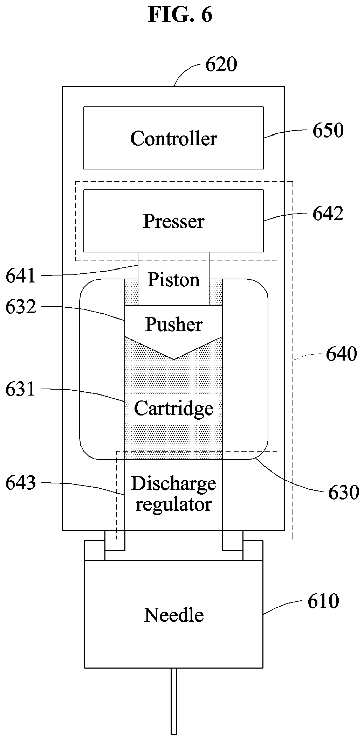

The driver 240 is configured to apply an external force to the medicine storage 230. For example, the driver 240 applies an external force to a back end of the medicine storage 230 such that the medicine is discharged through a fluid path formed from a front end of the medicine storage 230 to the needle 210. The driver 240 will be described in greater detail with reference to FIGS. 5 and 6.

The controller 250 is configured to measure an amount of the target analyte based on the electrical signal received through the first connector. When the measuring is completed, the controller 250 is configured to determine an injection amount of the medicine to be injected, and control the driver 240 to inject the determined injection amount. For example, the controller 250 may sense an electrical signal indicating an amount of a target analyte through a connector, and determine the amount of the target analyte from the sensed electrical signal. The controller 250 controls the driver 240 such that a precise amount of the medicine is discharged from the medicine storage 230 to the needle 210.

The notifier 260 is configured to receive, from the controller 250, measurement information associated with an amount of the target analyte and injection information associated with the injection amount, and provide a user with the received information. The notifier 260 may provide the user with the measurement information and the injection information through various means. The notifier 260 may include a sound outputter and a display, for example.

The sound outputter may output a beep sound and a voice sound that notify the user of the measurement information and the injection information. For example, the sound outputter may output, in a form of voice, a content indicating a measured blood glucose level and an injection amount of a medicine. For example, when the needle 210 is previously used once, the controller 250 may receive an error code from the needle 210, and the controller 250 may output a warning sound in a form of beep sound in response to the error code being received.

The display may visualize, for example, text information and sign information indicating the measurement information and the injection information, through graphic representation.

In addition, the notifier 260 may further include an optical outputter disposed on an outer circumferential surface of the housing 220. The optical outputter may include a light-emitting diode (LED), for example. For example, the optical outputter may output a content indicating a measured blood glucose level and an injection amount of a medicine through a color of light to be emitted from the LED, a sign to be indicated by the LED, or a blink of the LED (e.g., the number of blinks, a time length, and the like). The optical outputter may output light of a color matching a color of the cartridge to be mounted in the medicine injection device 200. Thus, when the user inserts in advance a type of a cartridge prescribed by a medical practitioner or specialist into the medicine injection device 200, the user may intuitively learn about the type of the cartridge inserted in the medicine injection device 200 from a color of light emitted.

The needle 210 is configured to invade skin, and collect the target analyte and discharge the medicine during the invasion into the skin. The needle 210 is configured to be connected to the medicine injection device 200, and includes an invasive member and the second connector. The invasive member is supported by the second fastener, and one end of the invasive member, for example, a front end, is formed to be invasive into the skin. In addition, inside the invasive member, a path through which a fluid moves along a longitudinal axis of the invasive member is formed. The second fastener may be a fastener configured to be connected to the housing 220 of the medicine injection device 200 through a structure that may support the invasive member, for example, an invasive suction member and an invasive injection member, and be attachable to or detachable from the medicine injection device 200. Hereinafter, the needle 210 will be described in greater detail with reference to FIGS. 3 and 4.

FIGS. 3 and 4 are diagrams illustrating examples of a configuration of a needle according to an example embodiment.

Referring to FIG. 3, a needle 310 is embodied in a structure configured to measure a target analyte and inject a medicine through a single invasion. As illustrated, the needle 310 includes an invasive injection member 311, an invasive suction member 312, and a second fastener 313.

The invasive injection member 311 is configured to invade skin and discharge a medicine. For example, as illustrated, a front end of the invasive injection member 311 is formed to invade the skin, and a back end of the invasive injection member 311 is configured to be connected to a medicine storage 330 when a header of a medicine injection device and the needle 310 are connected to each other.

The invasive suction member 312 is configured to invade the skin and absorb a body fluid sample. For example, as illustrated, a front end of the invasive suction member 312 is formed to invade the skin. The invasive suction member 312 is configured to absorb the body fluid sample through a capillarity phenomenon and a negative pressure applied from a back end thereof.

The second fastener 313 is configured to support the invasive injection member 311 and the invasive suction member 312, and be connected to the header. A second connector 319 configured to transfer an electrical signal to the medicine injection device is disposed on outer surface of the second fastener 313. When the header and the needle 310 are connected to each other, the second connector 319 of the needle 310 is configured to be connected to a first connector 321 disposed on an outer surface of the header to establish an electrical connection between the needle 310 and the medicine injection device. Similarly, when the header and the needle 310 are connected to each other, the first connector 321 of the medicine injection device is configured to be connected to the second connector 319 disposed on an outer surface of the needle 310 to establish the electrical connection between the needle 310 and the medicine injection device. The first connector 321 is disposed on the outer surface of the header. The first connector 321 and the second connector 319 may be a metal electrode, but not limited thereto.

For example, the second fastener 313 may be embodied in a fastening structure such as, for example, a screw structure and a magnetic structure. When a first fastener and the second fastener 313 are fastened to each other, the second connector 319 disposed on the second fastener 313 and the first connector 321 disposed on the first fastener are connected to each other.

For example, the first fastener and the second fastener 313 may be embodied in a thread structure and a thread groove structure. In this example, when a screwing connection is completed, the first connector 321 may be disposed at a position on an outer circumferential surface of the header at which the first connector 321 and the second connector 319 are electrically connectable. Similarly, when the screwing connection is completed, the second connector 319 may be disposed at a position on the outer surface of the needle 310 at which the first connector 321 and the second connector 319 are electrically connectable.

For another example, the first fastener and the second fastener 313 may be embodied by magnets. In this example, the magnets having different magnetic properties may be disposed at positions facing each other of the first fastener and the second fastener 313. Thus, for a magnetic connection, the magnets may be disposed such that the first connector 321 disposed on the first fastener and the second connector 319 disposed on the second fastener 313 are connected to each other. Here, a magnetic force of a magnetic property may be greater than a minimum magnetic force, and less than a maximum magnetic force. The maximum magnetic force may be less than a force induced by a human being, for example, a user, to remove the needle 310. The minimum magnetic force may be greater than a force of pulling the needle 310 by a living body and skin being invaded by the needle 310 while a medicine is being injected.

The needle 310 has a biosensor 314 configured to measure an amount of the target analyte, for example, a level of the target analyte. The biosensor 314 may be a sensor configured to convert an analyte to a signal. For example, the biosensor 314 may be a transducer configured to convert an amount of an analyte to a corresponding electrical signal in reaction to the analyte. An example of the biosensor 314 including enzyme that reacts to blood glucose in blood to trigger an electrochemical reaction will be mainly described herein. A portion onto which the enzyme is applied is referred to as the biosensor 314 herein. However, the biosensor 314 is not limited thereto.

In the example of FIG. 3, illustrated is a structure in which the biosensor 314 is formed in a space in the second fastener 313, while the needle 310 includes the two invasive members.

In this example, the second fastener 313 includes a sensing portion and the second connector 319. The biosensor 314 is formed in the sensing portion. The biosensor 314 is configured to sense a target analyte included in a body fluid sample absorbed through the invasive suction member 312. The sensing portion refers a space in which the body fluid sample is obtained, and onto which enzyme is applied. The medicine injection device is configured to generate a pressure from a front end of the invasive suction member 312 towards a back end of the invasive suction member 312, and thus transfer the body fluid sample from the front end of the invasive suction member 312 to the back end of the invasive suction member 312. For example, the medicine injection device may apply a negative pressure to the back end of the invasive suction member 312. In addition, when the invasive suction member 312 invades the skin, the body fluid sample may be extracted through a capillary phenomenon. The body fluid sample may be, for example, an interstitial fluid, but not limited thereto.

The second connector 319 is disposed on the outer surface of the second fastener 313 to be connectable to the first connector 321, and configured to be electrically connected to the biosensor 314. For example, the second connector 319 is configured to be electrically connected to the biosensor 314 and transfer an electrical signal generated in the biosensor 314 to the controller 350. In this example, the electrical signal measured in the sensing portion is transferred to the controller 350 of the medicine injection device through the second connector 319 and the first connector 321.

However, the biosensor 314 is not limited to being formed in the second fastener 313. In another example, the invasive suction member 312 includes the biosensor 314 configured to sense a target analyte included in a body fluid sample. In this example, the biosensor 314 is formed in the invasive suction member 312. For example, when the body fluid sample is absorbed into the invasive suction member 312 through a capillary phenomenon, the biosensor 314 formed in the invasive suction member 312 reacts to the target analyte and generates an electrical signal. In this example, the second fastener 313 includes the second connector 319. For example, the second connector 319 is configured to be electrically connected to the biosensor 314, and transfer the electrical signal generated in the biosensor 314 to the controller 350. The electrical signal generated by the biosensor 314 formed in the invasive suction member 312 is transferred to the controller 350 through the second connector 319 and the first connector 321.

In the example of FIG. 3, a second length 392 by which the invasive suction member 312 protrudes from the second fastener 313 is greater than a first length 391 by which the invasive injection member 311 protrudes from the second fastener 313. This is because a depth from which a bio-sample is extracted from skin is greater (or deeper) than a depth into which a medicine is injected. Such difference in length may minimize an immune effect in skin, and minimize a physical resistance that may occur in a living body adjacent to the needle 310 while blood glucose is being sensed. However, a length by which each of the invasive members is exposed from the second fastener 313 is not limited to the illustrated example.

In addition, at least one of the invasive suction member 312 or the invasive injection member 311 is configured to be slidable along one axis with respect to the second fastener 313. Thus, a length by which the invasive suction member 312 and the invasive injection member 311 are exposed from the second fastener 313 is configured to be adjustable.

Further, a diameter, a width, or a thickness of the invasive injection member 311 and the invasive suction member 312 may vary according to a design. For example, respective internal diameters and thicknesses of the invasive injection member 311 and the invasive suction member 312 may be the same, but not limited thereto. Alternatively, the invasive injection member 311 and the invasive suction member 312 may have different internal diameters and thicknesses.

In the example of FIG. 4, illustrated is a structure in which a biosensor is formed in an invasive member of a needle including two invasive members.

Referring to FIG. 4, a needle 410 includes one or more invasive members, for example, an invasive injection member 411 and an invasive suction member 412. One of the invasive members 411 and 412, for example, the invasive suction member 412, includes a biosensor 414 configured to sense a target analyte included in a body fluid sample. The biosensor 414 is formed in the invasive suction member 412. For example, the body fluid sample is directly diffused through enzyme of the biosensor 414 formed in the invasive suction member 412, and the biosensor 414 generates an electrical signal thereby in response to a reaction between the target analyte and the enzyme. Although FIG. 4 illustrates the needle 410 including the two invasive members 411 and 412, the number of invasive members is not limited to the illustrated example and may vary according to a design.

In the example of FIG. 4, a second fastener 413 includes a second connector 419. The second connector 419 is disposed on an outer surface of the second fastener 413 to be connectable to the first connector 321. In addition, the second connector 419 is configured to be electrically connected to the biosensor 414. For example, the second connector 419 is configured to be electrically connected to the biosensor 414 and transfer an electrical signal generated in the biosensor 414 to the controller 350. The electrical signal generated by the biosensor 414 formed in the invasive suction member 412 is transferred to the controller 350 through the second connector 419 and the first connector 321.

In another example, instead of a biosensor, for example, the biosensors 314 and 414 of FIGS. 3 and 4, being embodied directly in a needle, for example, the needles 310 and 410 of FIGS. 3 and 4, a test strip may be inserted into the needle. In this example, the test strip may be a blood glucose strip, for example. The blood glucose strip may be a test strip that is chemically prepared to be smeared with blood. When the test strip is inserted into an insertion hole of the needle, one end of the inserted test strip on which enzyme is applied may be connected to a back end of an invasive suction member of the needle, for example, the invasive suction members 312 and 412 of FIGS. 3 and 4. At another end of the test strip, an electrode configured to transfer an electrical signal generated by an enzyme reaction may be formed. The electrode may be connected to a second connector of the needle, for example, the second connectors 319 and 419 of FIGS. 3 and 4, or a first connector of a medicine injection device, or form an electrical connection to a controller through other structures.

FIGS. 5 and 6 are diagrams illustrating examples of a configuration of a driver according to an example embodiment.

As described above, a medicine storage may accommodate a cartridge configured to store a medicine. A driver is configured to apply an external force to a pusher of the cartridge accommodated in the medicine storage. Hereinafter, examples of how the driver applies an external force to the pusher of the cartridge will be described in detail with reference to FIGS. 5 and 6.

FIG. 5 illustrates a mechanical structure configured to directly move a pusher forward under the control of a controller.

Referring to FIG. 5, in response to a control operation of a controller 550, a driver 540 moves a piston 541 of a cartridge 531 accommodated in a medicine storage 530 from a back end of a medicine injection device to a front end of the medicine injection device by a distance corresponding to a determined injection amount of a medicine. In an example, the driver 540 includes a motor and a linear gear. The motor is configured to rotate a shaft by a rotation amount determined by the controller 550. The linear gear is engaged with a gearbox to be connected to the shaft of the motor. In this example, when the motor rotates the shaft, the linear gear moves forward from the back end of the medicine injection device to the front end of the medicine injection device along a longitudinal axis of the medicine injection device. When an injection signal indicating an injection instruction is generated by a user, the controller 550 allows the linear gear to move forward by a length corresponding to the determined injection amount. A front end of the linear gear is connected to a back end of the piston 541, and a front end of the piston 541 is connected to a back end of the pusher 532. Thus, the controller 550 controls a distance by which the pusher 532 moves forward by regulating a rotation of the motor. The controller 550 controls the driver 540 to allow the piston 541 and the pusher 532 to move forward by the distance corresponding to the determined injection amount such that the determined injection amount of the medicine is discharged to a needle 510 connected to the medicine injection device through a second fastener 519 of the needle 510 and a first fastener 529 of a housing 520.

FIG. 6 illustrates a structure configured to regulate an amount of a medicine to be discharged from a front end of a medicine storage under the control of a controller.

Referring to FIG. 6, a driver 640 includes a presser 642 and a discharge regulator 643.

The presser 642 is configured to apply an external force to a front end of a medicine injection device from a back end of the medicine injection device along a longitudinal axis of the medicine injection device. For example, the presser 642 is embodied as an elastic member fixed inside a housing 620. In this example, one end of the elastic member transfers the external force to a piston 641 in a direction from a back end of the housing 620 towards a front end of the housing 620. In addition, the piston 641 is configured to be connected to a pusher 632, and thus the pusher 632 of a cartridge 631 accommodated in a medicine storage 630 receives a pressure by the presser 642. That is, the presser 642 transfers the external force to the pusher 632 through the piston 641, and thus generates a pressure that enables a medicine stored in the medicine storage 630 to be discharged to a front end of the medicine storage 630.

In response to the pressure generated by the presser 642, the discharge regulator 643 is configured to regulate a fluid flow such that a desirable or optimal amount of the medicine is transferred to a needle 610 under the control of a controller 650. The discharge regulator 643 is configured to regulate a discharge amount of the medicine to be discharged to the needle 610 connected to a header from the medicine storage 630 based on a determined injection amount of the medicine. The discharge regulator 643 may be embodied as, for example, a micropump and a piezoelectric pump that regulates an amount of the medicine to be transferred from the medicine storage 630 to the needle 610. The discharge regulator 643 is configured to regulate a pressure and the like, and discharge a precise amount of the medicine to the needle 610 from the cartridge 631.

When an injection signal indicating an injection instruction is generated by a user, the controller 650 controls the discharge regulator 643 disposed at a front end of the cartridge 631 to regulate the discharge amount of the medicine by a precise unit.

In an example, the structure illustrated in FIG. 6 may be embodied in a form of a box. When the medicine injection device is embodied in a form of a box, a surface area in which skin and the housing 620 are in contact during an invasion of the needle 610 into the skin may be relatively great, and thus shaking or instability may be reduced and a relatively high level of safety or stability may be achieved. In addition, such a box form may increase a form factor, and thus the medicine injection device may minimize an influence of a temperature due to an internal and/or external factor. Further, the medicine injection device may be manufactured through a simpler or more streamlined process.

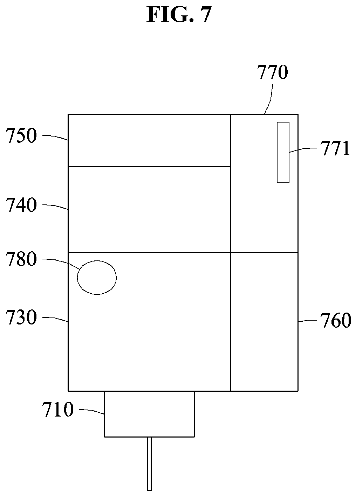

FIG. 7 is a diagram illustrating an example of an additional configuration of a medicine injection device according to an example embodiment.

Referring to FIG. 7, a housing is configured to accommodate a medicine storage 730, a driver 740, a controller 750, and a notifier, and be connected to a needle 710 through a header thereof. The housing further includes a communicator 760, a battery 770, and a temperature measurer 780.

The temperature measurer 780 is disposed separate from the battery 770, and configured to measure a temperature. Thus, the temperature measurer 780 may minimize an influence of the battery 770 of which temperature may increase due to charging of the battery 770.

The communicator 760 is configured to establish communication with an external server and a mobile terminal. For example, the communicator 760 may establish wireless communication with the mobile terminal, and establish communication with the external server, for example, a cloud server, via the mobile terminal. In this example, the communicator 760 may transmit measurement information (associated with a living body) to the external server and receive injection information from the external server, via the mobile terminal.

The battery 770 is configured to be charged using electrical power to be supplied through a terminal 771, for example, a 5-pin terminal and a C-type terminal. In addition, the controller 750 is configured to exchange, or receive and transmit, data through the terminal 771. Hereinafter, a method of controlling an amount of power stored in the battery 770, or a state of charge (SOC) of the battery 770, will be described.

In an example, in response to an SOC of the battery 770 being less than or equal to a reference threshold, the controller 750 may provide a user with information that is an available number of injections through the notifier using at least one of a sound signal or a visual signal. The controller 750 may output a maximum available number of injections based on a time of use through a color of an LED and a sign, and the number of blinks of the LED, or in a form of a sound and a text. In addition, the controller 750 may reserve or store backup power of the SOC for at least one injection. Thus, in response to an emergency injection instruction being received from the user, the controller 750 may trigger an injection operation using the reserved backup power.

In this example, in response to the SOC of the battery 770 exceeding the reference threshold while external power is being supplied through a power source socket of a medicine injection device, the controller 750 may allow an injection during charging. Thus, even when the battery 770 is charged with a certain amount of power or greater during the charging, the controller 750 may allow the injection operation. The reference threshold may be a value used to be a reference for determining whether to charge or not the battery 770. That the SOC of the battery 770 is less than or equal to the reference threshold may indicate a less amount of power stored or remained in the battery 770. Thus, in such case, the controller 750 may provide, to the user through the notifier, information about a lack of the SOC of the battery 770 and a remaining number of injections, and thus the user may be informed of a need to charge the battery 770.

In response to the SOC of the battery 770 being less than or equal to the reference threshold and exceeding a minimum threshold, while the external power is being supplied through the power source socket of the medicine injection device, the controller 750 may allow an injection during the charging. Thus, when the battery 770 is charged with power for at least one injection, the controller 750 may allow an injection operation even though the SOC of the battery 770 is relatively small during the charging. Here, when the SOC of the battery 770 is less than or equal to the reference threshold, the controller 750 may calculate an available number of injections with current remaining power, or a current SOC, and notify the user of the calculated available number of injections. The minimum threshold may indicate an amount of power to be consumed by the medicine injection device for one injection operation.

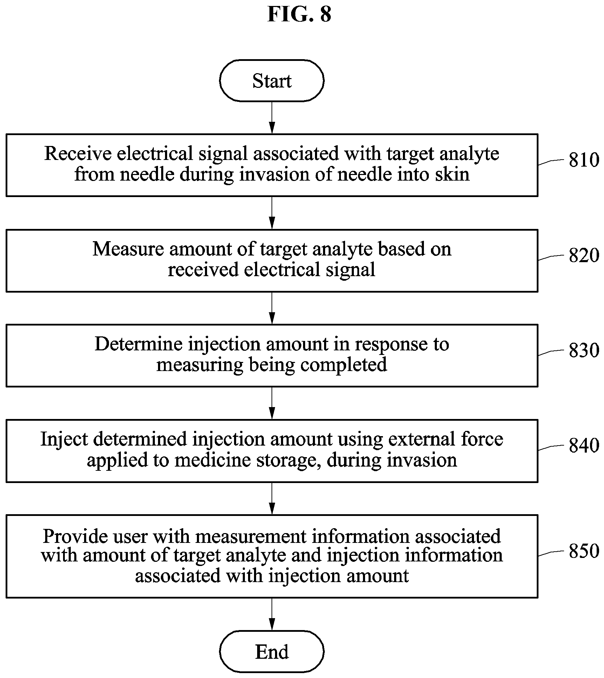

FIG. 8 is a flowchart illustrating an example of a medicine injection method according to an example embodiment.

Referring to FIG. 8, in operation 810, a medicine injection device receives, from a needle, an electrical signal associated with a target analyte during an invasion of the needle into skin. For example, as described above, when the needle invades the skin, the needle absorbs a body fluid sample through a capillary phenomenon. The needle is configured to extract the body fluid sample by an amount required for sensing, and transfer the extracted body fluid sample to a biosensor. The biosensor may be formed in the needle, but not limited thereto. The biosensor may also be formed in a header of the medicine injection device or a portion adjacent to the header. The biosensor is configured to be connected to an invasive suction member of the needle, and receive the body fluid sample absorbed by the needle. The biosensor is configured to react to the target analyte in the body fluid sample, and generate the electrical signal. The medicine injection device then receives the electrical signal from the needle through a connector.

In operation 820, the medicine injection device measures an amount of the target analyte based on the received electrical signal. The medicine injection device obtains a digital value indicating the measured amount of the target analyte by performing analog-to-digital conversion (ADC) on the electrical signal generated by the biosensor. The medicine injection device determines final measurement information associated with the target analyte, or a living body, by adjusting or correcting the digital value. Here, the measurement information may be a blood glucose level, for example.

In operation 830, when the measuring is completed, the medicine injection device determines an injection amount of a medicine to be injected. In an example, when the final measurement information is determined, the medicine injection device may determine that the measuring is completed. The medicine injection device determines the injection amount based on the measurement information. For example, the medicine injection device may determine an insulin injection amount based on a measured blood glucose level using various algorithms associated with a blood glucose level and an insulin injection amount. In this example, the medicine injection device may refer to data transferred from a server, and calculate an amount of a medicine to be injected based on a measured glucose level using a certain algorithm. For another example, the medicine injection device may transmit the measurement information to the server, and receive information about an amount of a medicine to be injected from the server.

In operation 840, the medicine injection device injects the determined injection amount of the medicine using an external force applied to a medicine storage of the medicine injection device, during the invasion. For example, the medicine injection device may discharge the medicine by a unit of the determined injection amount by controlling a driver of the medicine injection device as described above with reference to FIGS. 5 and 6.

In operation 850, the medicine injection device provides a user with the measurement information associated with the amount of the target analyte and injection information associated with the injection amount. For example, the medicine injection device may output the measurement information and the injection information in a form of a sound, or visualize the measurement information and the injection information in a form of a text or a sign. In this example, the medicine injection device may output a sound signal indicating a blood glucose level, or output a text indicating a blood glucose level to a display. In addition, the medicine injection device may output a sound signal indicating a time at which insulin is administered and an administered amount of insulin, or output a text indicating an injection amount to the display.

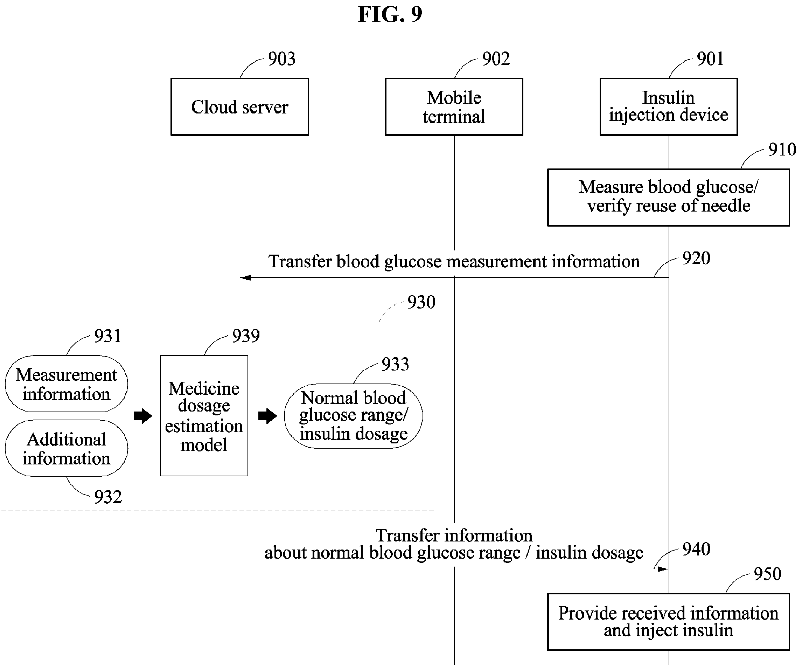

FIG. 9 is a diagram illustrating an example of a medicine injection method according to an example embodiment.

In the example of FIG. 9, a medicine injection device is designed to receive an insulin cartridge to be mounted therein, and is also referred to as an insulin injection device 901.

Referring to FIG. 9, in operation 910, the insulin injection device 901 generates measurement information associated with a living body or a target analyte, and verifies whether a needle is previously used or not. For example, the insulin injection device 901 may verify whether the needle connected to a header of the insulin injection device 901 is previously used, and prevent an injection using the needle when the needle is verified to be previously used. The insulin injection device 901 may inform a user that the needle is used once by providing a warning sound and the like through a notifier. Thus, the insulin injection device 901 may prevent reuse of the needle. When the needle invades skin, the insulin injection device 901 measures an amount of a target analyte from a body fluid sample collected through the needle, and generates measurement information associated with the amount of the target analyte, for example, a blood glucose level.

In operation 920, the insulin injection device 901 transfers, to a cloud server 903, the measurement information associated with a blood glucose level. For example, when the measuring is completed, the insulin injection device 901 transfers the measurement information to the cloud server 903 through a communicator. The cloud server 903 stores the measurement information, for example, measurement information 931 as illustrated. The measurement information 931, which is associated with measuring a target analyte, may include, for example, a user index (e.g., user identifier [ID]), a measured blood glucose level, a measurement time, and other information associated with the target analyte. In addition, the insulin injection device 901 generates additional information 932 and transfers the generated additional information 932 to the cloud server 903. The additional information 932 may include, for example, information about a type of a medicine provided to the insulin injection device 901, for example, insulin, and a temperature at a measurement time, and device information of the insulin injection device 901 and a mobile terminal 902. The device information may include hardware specification of a corresponding device. The insulin injection device 901 may establish wireless communication, for example, WiFi, Bluetooth, and Zigbee, with the mobile terminal 902, and the mobile terminal 902 may establish a communication connection to the cloud server 903. Thus, the insulin injection device 901 may transfer the measurement information 931 to the cloud server 903 via the mobile terminal 902.

In operation 930, the cloud server 903 calculates injection information 933 from the measurement information 931 using a medicine dosage estimation model 939. For example, the medicine dosage estimation model 939 may be a model configured to calculate the injection information 933 using a predefined function from a plurality of sets of input data. In this example, the sets of input data may include, for example, the measurement information 931 and the additional information 932.

For another example, the medicine dosage estimation model 939 may be a machine learning structure configured to output an injection amount of a medicine to be administered from the measurement information 931, and include a neural network, for example. The medicine dosage estimation model 939 may be trained to output a reference injection amount corresponding to a reference blood glucose level. In addition, the medicine dosage estimation model 939 may receive the measurement information 931 and the additional information 932, and output the injection information 933 therefrom. The injection information 933 may include, for example, a normal blood glucose range and an insulin dosage to be administered. The medicine dosage estimation model 939 may be trained to more accurately calculate the normal blood glucose range and the insulin dosage by receiving various sets of information.

In operation 940, the cloud server 903 transfers the calculated information, for example, the normal blood glucose range and the insulin dosage, to the insulin injection device 901. The insulin injection device 901 receives the injection information 933 corresponding to the measurement information 931 from the cloud server 903, and determines an injection amount based on the received injection information 933. The normal blood glucose range may include a reference range used to verify whether a measured blood glucose level is an allowable or tolerable level. The insulin dosage may indicate an injection amount of a medicine or insulin that needs to be administered or injected to the user at a current point in time.

In operation 950, the insulin injection device 901 provides the user with the received information, and injects insulin. For example, the insulin injection device 901 may inform the user of whether a current blood glucose level of the user is in the normal range or out of the normal range based on the information about the normal blood glucose range that is received from the cloud server 903. In this example, when an injection of insulin is required, the insulin injection device 901 may inject the insulin by an injection amount indicated in the injection information 933 determined by the cloud server 903.

In an example, the insulin injection device 901 may provide the user with a direct treatment, for example, an injection of insulin, in addition to measuring a blood glucose level of the user. That is, a time at which a blood glucose level is measured by the insulin injection device 901 and a time at which insulin is injected by the insulin injection device 901 may be the same. In addition, the insulin injection device 901 may preclude reuse of the needle when there is an attempt to reuse the needle. Since the insulin injection device 901 measures blood glucose and injects insulin simultaneously during a single invasion, there is no need to adjust or correct an error that may occur due to an immune reaction when measuring the blood glucose. In addition, the insulin injection device 901 may include two invasive members in the needle configured to inject a medicine and measure blood glucose, and the two invasive members may invade skin simultaneously or successively. The insulin injection device 901 may inject, readily and accurately, an automatically determined amount of insulin through an electronic button that is different from an existing means for injecting insulin, for example, a dial and a mechanical button.

According to example embodiments described herein, a medicine injection device may measure an amount of a target analyte and inject a medicine, through a single invasion.

According to example embodiments described herein, a medicine injection device may have no gap between a time to measure a target analyte and a time to inject a medicine.

According to example embodiments described herein, a medicine injection device may automatically and accurately set a dosage of a medicine to be administered, and more finely change or regulate an injection amount of the medicine.

According to example embodiments described herein, a medicine injection device may inject a medicine stably through a housing with a relatively great size of a contact area being in contact with skin.

According to example embodiments described herein, a medicine injection device may automatically record information associated with an injection of a medicine.

According to example embodiments described herein, a medicine injection device may remotely receive a prescription or remotely upload an analyte measurement history and a medicine injection history, without a visit to a hospital each time.

According to example embodiments described herein, a medicine injection device may facilitate replacement of a needle.

According to example embodiments described herein, a medicine injection device may effectively prevent reuse of a needle.

According to example embodiments described herein, a medicine injection device may measure blood glucose and inject insulin simultaneously during a single invasion, and thus may not need to correct an error that may be caused by an immune reaction when measuring the blood glucose.

The units described herein may be implemented using hardware components and software components. For example, the hardware components may include microphones, amplifiers, band-pass filters, audio to digital convertors, non-transitory computer memory and processing devices. A processing device may be implemented using one or more general-purpose or special purpose computers, such as, for example, a processor, a controller and an arithmetic logic unit, a digital signal processor, a microcomputer, a field programmable array, a programmable logic unit, a microprocessor or any other device capable of responding to and executing instructions in a defined manner. The processing device may run an operating system (OS) and one or more software applications that run on the OS. The processing device also may access, store, manipulate, process, and create data in response to execution of the software. For purpose of simplicity, the description of a processing device is used as singular; however, one skilled in the art will appreciated that a processing device may include multiple processing elements and multiple types of processing elements. For example, a processing device may include multiple processors or a processor and a controller. In addition, different processing configurations are possible, such a parallel processors.

The software may include a computer program, a piece of code, an instruction, or some combination thereof, to independently or collectively instruct or configure the processing device to operate as desired. Software and data may be embodied permanently or temporarily in any type of machine, component, physical or virtual equipment, computer storage medium or device, or in a propagated signal wave capable of providing instructions or data to or being interpreted by the processing device. The software also may be distributed over network coupled computer systems so that the software is stored and executed in a distributed fashion. The software and data may be stored by one or more non-transitory computer readable recording mediums. The non-transitory computer readable recording medium may include any data storage device that can store data which can be thereafter read by a computer system or processing device. Examples of the non-transitory computer readable recording medium include read-only memory (ROM), random-access memory (RAM), CD-ROMs, magnetic tapes, floppy disks, optical data storage devices. Also, functional programs, codes, and code segments that accomplish the examples disclosed herein can be easily construed by programmers skilled in the art to which the examples pertain based on and using the flow diagrams and block diagrams of the figures and their corresponding descriptions as provided herein.

While this disclosure includes specific examples, it will be apparent to one of ordinary skill in the art that various changes in form and details may be made in these examples without departing from the spirit and scope of the claims and their equivalents. The examples described herein are to be considered in a descriptive sense only, and not for purposes of limitation. Descriptions of features or aspects in each example are to be considered as being applicable to similar features or aspects in other examples. Suitable results may be achieved if the described techniques are performed in a different order, and/or if components in a described system, architecture, device, or circuit are combined in a different manner and/or replaced or supplemented by other components or their equivalents.

Therefore, the scope of the disclosure is defined not by the detailed description, but by the claims and their equivalents, and all variations within the scope of the claims and their equivalents are to be construed as being included in the disclosure.

## Claims

1. A medicine injection device comprising:
a header including a first connector configured to receive an electrical signal associated with a target analyte from a needle, and configured to be connected to the needle;
a medicine storage configured to be connected to the needle when the needle and the header are connected to each other, and store a medicine;
a driver configured to apply an external force to the medicine storage;
a controller configured to control the driver to measure an amount of the target analyte based on the electrical signal received through the first connector, determine an injection amount of the medicine to be injected in response to the measuring being completed, and inject the determined injection amount; and
a notifier configured to receive, from the controller, measurement information associated with the amount of the target analyte and injection information associated with the injection amount, and provide a user with the received measurement information and injection information.

2. The medicine injection device of claim 1, wherein the first connector is disposed on an outer surface of the header, and configured to establish an electrical connection between the needle and the medicine injection device as the first connector is connected to a second connector disposed on an outer surface of the needle when the header and the needle are connected to each other.

3. The medicine injection device of claim 1, wherein the medicine storage is configured to accommodate a cartridge configured to store the medicine, and
the driver is configured to apply the external force to a pusher of the cartridge accommodated in the medicine storage.

4. The medicine injection device of claim 1, wherein the driver comprises:
a presser configured to apply the external force to a front end of the medicine injection device from a back end of the medicine injection device along a longitudinal axis of the medicine injection device; and
a discharge regulator configured to regulate, based on the determined injection amount, a discharge amount to be discharged to the needle connected to the header from the medicine storage.

5. The medicine injection device of claim 1, wherein the driver is configured to:
move a piston of a cartridge accommodated in the medicine storage from a back end of the medicine injection device to a front end of the medicine injection device by a distance corresponding to the determined injection amount, under the control of the controller.

6. The medicine injection device of claim 1, wherein the needle comprises:
an invasive suction member configured to invade skin and absorb a body fluid sample;
an invasive injection member configured to invade the skin and discharge the medicine; and
a second fastener configured to support the invasive suction member and the invasive injection member, and be connected to the header.

7. The medicine injection device of claim 6, wherein the invasive suction member includes a biosensor configured to sense the target analyte included in the body fluid sample,
wherein the invasive injection member is configured to be connected to the medicine storage when the header and the needle are connected to each other, and discharge the medicine to be transferred from the medicine storage, and
wherein the second fastener includes a second connector disposed on an outer surface of the second fastener to be connectable to the first connector and configured to be electrically connected to the biosensor.

8. The medicine injection device of claim 6, wherein the second fastener includes:
a sensing portion including a biosensor configured to sense the target analyte included in the body fluid sample absorbed through the invasive suction member; and
a second connector disposed on an outer surface of the second fastener to be connectable to the first connector, and configured to be electrically connected to the biosensor.

9. The medicine injection device of claim 6, wherein at least one of the invasive suction member or the invasive injection member is configured to be slidable along one axis with respect to the second fastener.

10. The medicine injection device of claim 6, wherein a second length by which the invasive suction member protrudes from the second fastener is greater than a first length by which the invasive injection member protrudes from the second fastener.

11. The medicine injection device of claim 1, wherein the controller is configured to:
when the needle invades skin, generate measurement information by measuring an amount of a target analyte from a body fluid sample collected through the needle;
when the measuring is completed, transfer the measurement information to a cloud server through a communicator;
receive injection information corresponding to the measurement information from the cloud server; and
determine the injection amount based on the received injection information,
wherein the injection information is calculated from the measurement information by the cloud server based on a medicine dosage estimation model.

12. The medicine injection device of claim 1, wherein the controller is configured to:
verify whether the needle connected to the medicine injection device is previously used; and
when the needle is verified to be previously used, prevent an injection using the needle.

13. The medicine injection device of claim 1, wherein the controller is configured to:
when a state of charge (SOC) of a battery is less than or equal to a reference threshold, provide the user with information that is an available number of injections through the notifier using at least one of a sound signal, a visual signal, a vibration signal, or a tactile signal, and reserve backup power of the SOC for at least one injection;
when an SOC of a battery exceeds a reference threshold while external power is being supplied through a power source socket of the medicine injection device, allow an injection during charging of the battery; and/or
when an SOC of a battery is less than or equal to a reference threshold and exceeds a minimum threshold while external power is being supplied through a power source socket of the medicine injection device, allow an injection during charging of the battery.

14. The medicine injection device of claim 1, further comprising:
a housing configured to accommodate the medicine storage, the driver, the controller, and the notifier, and configured to be connected to the needle through the header.

15. A medicine injection method comprising:
receiving an electrical signal associated with a target analyte from a needle during an invasion of the needle into skin;
measuring an amount of the target analyte based on the received electrical signal;
determining an injection amount in response to the measuring being completed;
during the invasion, injecting the determined injection amount using an external force applied to a medicine storage; and
providing a user with measurement information associated with the amount of the target analyte and injection information associated with the injection amount.
